# EUROPEAN PATENT SPECIFICATION

(11) **EP 2 346 520 B1**
(45) Date of publication and mention of the grant of the patent: **26.11.2014**
(21) Application number: 09833074.9
(22) Date of filing: 17.11.2009
(51) Int. Cl.: A61K 31/12, A61K 9/10, A61P 27/02

(54) **CURCUMINOIDS AND ITS METABOLITES FOR THE APPLICATION IN OCULAR DISEASES**
CURCUMINOIDE UND IHRE STOFFWECHSELPRODUKTE ZUR ANWENDUNG BEI AUGENERKRANKUNGEN
CURCUMINOÏDES ET LEURS MÉTABOLITES POUR DES APPLICATIONS RELATIVES AUX MALADIES OCULAIRES

(30) Priority: 17.11.2008 IN CH28272008
(43) Date of publication of application: 27.07.2011
(73) Proprietor: Laila Pharmaceuticals Pvt. Ltd., Chennia 600 050 (IN)
(72) Inventor: CHANIYILPARAMPU, Ramchand, Nanappan, Chennai 600 037 (IN); NAIR, Anitha, Krishnan, Chennai 600 050 (IN); KAPOOR, Anuj, Chennai 600 050 (IN); PARTHASARATHY, Kavitha, Chennai 600 050 (IN); KIRAN, Bhupathiraju, Chennai 600 037 (IN); GOKARAJU, Rama, Raju, Chennai 600 037 (IN); GOKARAJU, Ganga, Raju, Chennai 600 037 (IN); GOLAKOTI, Trimurtulu, Chennai 600 037 (IN)
(74) Representative: Rees, Kerry
(86) International application number: PCT/IN2009/000650
(87) International publication number: WO 2010/070664

(56) References cited:
- EP-A2- 1 103 266
- WO-A1-2010/010431
- WO-A2-2007/103435
- CN-A- 1 857 675
- GB-A- 2 415 902
- KR-A- 20020 084 336
- TOMREN ET AL: "Studies on curcumin and curcuminoids", INTERNATIONAL JOURNAL OF PHARMACEUTICS, ELSEVIER BV, NL, vol. 338, no. 1-2, 23 May 2007 (2007-05-23), pages 27-34, XP022093433, ISSN: 0378-5173, DOI: 10.1016/J.IJPHARM.2007.01.013
- LOFTSSON THORSTEINN ET AL: "CYCLODEXTRINS IN DRUG DELIVERY", EXPERT OPINION ON DRUG DELIVERY, INFORMA HEALTHCARE, GB, vol. 2, no. 2, 1 March 2005 (2005-03-01), pages 335-351, XP008075998, ISSN: 1742-5247, DOI: 10.1517/17425247.2.1.335
- TONNESEN HANNE HJORTH ET AL: "Studies of curcumin and curcuminoids. XXVII. Cyclodextrin complexation: Solubility, chemical and photochemical stability", INTERNATIONAL JOURNAL OF PHARMACEUTICS, ELSEVIER BV, NL, vol. 244, no. 1-2, 5 September 2002 (2002-09-05), pages 127-135, XP002473303, ISSN: 0378-5173, DOI: 10.1016/S0378-5173(02)00323-X

## Description

### Technical Field of the invention:

The present invention relates to an aqueous ophthalmic composition comprising natural curcuminoids, such as curcumin, bisdemethoxycurcumin, demethoxycurcumin and synthetically derived bis-o-demethyl curcumin and/or other demethylated curcuminoids either alone or in combinations, suitable surfactants and co-solvents as permeation enhancers along with other ophthalmic excipients, useful for the treatment of ocular diseases or disorders.

### Background of the invention:

Eye is a complex and sensitive organ of the body, which can experience numerous diseases, and other deleterious conditions that affect its ability to function normally and many such ocular conditions can be found in the interior and most particularly at the rear or posterior part of the eye.

Ocular condition is a disease or a condition associated with the eye or one of the parts or regions of the eye. Broadly this can be differentiated on the basis of their anatomy and the associated disease with the same as anterior ocular and posterior ocular condition. The anterior ocular condition includes an anterior (i.e. front of the eye) ocular region or site which is associated with astigmatism; blepharospasm; cataract; conjunctival diseases; conjunctivitis; corneal diseases; corneal ulcer; dry eye syndromes; eyelid diseases; lacrimal apparatus diseases; lacrimal duct obstruction; myopia; presbyopia; pupil disorders; refractive disorders and strabismus. A posterior ocular condition includes a posterior ocular region or site which include macular degeneration, Behcet's disease, retinal disorders, diabetic retinopathy (including proliferative diabetic retinopathy); retinal arterial occlusive disease; central retinal vein occlusion; uveitic retinal disease; retinal detachment; ocular trauma which affects a posterior ocular site or location.

The other major disease associated with eyes are glaucoma - an eye disease that damages the optic nerve and impairs vision (sometimes progressing to blindness), macular degeneration - caused by degeneration of the cells of the macula lutea and results in blurred vision; can cause blindness and retinopathy - a disease of the retina that can result in loss of vision. For prevention or cure of these disease drug can be administered through the most advantageous ocular drug delivery. However, the number of drugs used in ocular drug delivery system has been somewhat limited, in part resulting from the formidable barriers to drug permeation presented both by the upper layer of skin, the stratum corneum, and the cornea in the eye. The only drugs that could permeate these barriers are those possessing vary narrow and specific combination of physicochemical properties that can be achieved for example by microsphers or nanotechnology with an extended residence time and a target specific drug delivery. These systems were developed to overcome solubility problems of poorly soluble drugs.

Drug delivery to the posterior part of the eye is important for treating several disorders such as age-related macular degeneration, diabetic retinopathy, retinal venous occlusions, retinal arterial occlusion, macular edema, postoperative inflammation, uveitis retinitis, proliferative vitreoretinopathy and glaucoma. Due to anatomic membrane barriers (i.e. cornea, conjunctiva and sclera) and the lachrymal drainage it can be quite challenging to obtain therapeutic drug concentrations in the posterior parts of the eye after topical drug administration. These barriers cannot be altered with non-invasive methods; the ophthalmic formulations have to be improved in some way to increase the ocular bioavailability. To date, there is no noninvasive, safe and patient-friendly drug delivery system that is specific and effective for the posterior part of the eye. In most cases, topical or systemic administration is used to treat posterior diseases despite limited bioavailability from these formulations.

Presently, there has been a tremendous surge in demand for non-steroidal, plant based anti-inflammatory agents and their potential use in various therapeutic applications.

In nature, curcuminoids are of three types, a) Curcumin b) Bisdemethoxy curcumin c) Demethoxy curcumin, which are all polyphenols and are responsible for the yellow color of turmeric. The active Curcumin, is the principal curcuminoid out of the three an exceptionally potent antioxidant and anti-inflammatory, which can prevent both free-radical damage and inflammation which are the major factors of cell damage associated with oxidation or Oxidative stress. The current level of interest on curcumin and its analogs/derivatives is known from the below mentioned articles.

Process for producing enriched fractions of bis-o-demethylcurcumin and tetrahydrotetrahydroxy-curcumin from the extracts of *curcuma longa* is disclosed in WO/2007/043058, the contents of which are incorporated herein in its entirety. According to this publication, bis-o-demethyl curcumin shows most potent anticancer, antioxidative and inflammatory activity when compared to other curcuminoids and the natural mixture of curcumins.

An article on studies of curcumin and curcuminoids XXXI; Symmetric and asymmetric curcuminoids, stability, activity and complexation with cyclodextrin by Thorsteinn Loftson et al in International Journal of pharmaceutics vol 338, issues 1-2, pages 27-34.

Further, an article on beta cyclodextrin inclusion complex of curcumin by Beena Mistra et al, is described in BARC news letter, India; issue no 185, October 2007.

However, the use of natural curcuminoids, such as curcumin, bisdemethoxycurcumin demethoxycurcumin and synthetically derived bis-o-demethyl curcumin and/or other demethylated curcuminoids either alone or in combination in ophthalmic formulations is not known in the prior art. Natural curcuminoids, such as curcumin, bisdemethoxycurcumin demethoxycurcumin and synthetically derived bis-o-demethyl curcumin and/or other demethylated curcuminoids either alone or in combination being a lipophilic drugs have low aqueous solubility, and hence restricts the therapeutic availability of the same.

All curcuminoids, both natural and synthetic have lipophilic structures with conjugated double bonds with a keto group thus rendering them to have a dielectric constant which will interact with negatively charged membranes making it unable to permeate through the biological membrane. Thus, for these curcuminoids to pass through membranes, it is necessary to encapsulate these molecules with lipophilic molecules. Moreover, it is necessary to make the lattice of molecules as a single entity so that these encapsulated molecules can be transported across membranes.

"Cyclodextrins" are crystalline, homogenous and non-hygroscopic cyclic oligosaccharides consisting of multiple (a, D1-4) linked glucopyranose units that display amphoteric properties of a lipophilic central cavity and hydrophilic outer surface, which have been in use over past decade, to increase the solubility of a lipophillic drug. In aqueous solutions cyclodextrins are able to form inclusion complexes with many drugs by taking up a drug molecule or more frequently some lipophilic moiety of the molecule, into the central cavity. This property has been extensively utilized by the drug formulators for drug delivery purpose to increase the bioavailability of the drugs having limited oral bioavailability due to poor dissolution rate and solubility by improving their absorption. This complexation further reduces active recrystalization of drugs, helps to increase their aqueous solubility.

It has been shown that complexation efficacy of cyclodextrins can be significantly enhanced by including small amount of a water- soluble polymer in the aqueous complexation medium as the water-soluble polymers improve both pharmaceutical and biological properties of drug/cyclodextrin complexes. However, it has been further shown that cyclodextrins and cyclodextrin complexes self-associate to form aggregates and that those aggregates can act as solubilizers themselves.

Further, newer technology like microspher or nanotechnology with cyclodextrin complex and an active ingredient with wide pharmacologically activity with less toxic effect can have of first choice to administer via ocular route

Ophthalmic drugs are classified into analgesics, antihistamines, antimicrobials, corticosteroids and non steroidal anti inflammatory drugs. These classes of drugs used to treat variety of ocular disorders. Prolonged use of corticosteroids, NSAID's , and the other groups of above mentioned classes of drugs results in glaucoma, optic nerve damage, results in vision problems, cataracts, or secondary ocular infections.

Accordingly, the current invention aims to provide a potential successor in the field of ophthalmology with a novel herbal composition for the treatment of ocular diseases/disorders. Surprisingly, preliminary experiments conducted have demonstrated the use of non-ionic surfactants and co-solvents in solubilizing curcuminoids upto 200mg thereby aiding effective solubilization in aqueous medium. The inventors have found that coating of curcuminoidds using non-ionic surfactants and a cosolvent enhances the curcuminoid's aqueous solubility.

Therefore, the current invention provides an aqueous ophthalmic composition comprising; a) natural or synthetic curcuminoid(s) either alone or in combination in an amount of 0.02 to 2.5%w/v wherein the particle size of curcuminoid is present in the range of 8-11 nm (nanometer); b) nonionic surfactant(s) in an amount of 1%w/v; c) and a polyhydric alcohol co-solvent(s) in an amount of 1%w/v along with pharmaceutically acceptable ophthalmic excipients, for increasing the bioavailability of the active compounds when administered topically to the eye.

In a further aspect the present invention provides the aqueous ophthalmic composition as claimed in any one of Claims 1 to 7 for use in the prevention and treatment of ocular disease conditions including Behcet's disease, retinal disorders, diabetic retinopathy, cataract, conjunctival diseases, corneal diseases including macular degeneration and glaucoma.

### Description of Drawings:

**Figure-1:** The dose response curve indicating a concentration dependent increase in the transport of nanoemulsified LI01008 across A549 cell monolayers
**Figure-2:** The graph indicating the size of nano-emulsified curcumin 98%.
**Figure-3:** The graph indicating the size of unformulated curcumin 98%.
**Figure-4:** The graph indicating the size of nano-emulsified BDMC.

### Summary of the Invention:

The instant invention provides an aqueous ophthalmic (topical) eye drop or eye gel formulation comprising natural curcuminoids, such as curcumin, bisdemethoxycurcumin demethoxycurcumin and synthetically derived bis-o-demethyl curcumin and/or other demethylated curcuminoids either alone or in combinations together with nonionic surfactant, cosolvent along with suitable ophthalmic excipients.

In another aspect, the invention provides an aqueous ophthalmic eye drop or eye gel formulation comprising natural curcuminoids, such as curcumin, bisdemethoxycurcumin demethoxycurcumin and synthetically derived bis-o-demethyl curcumin and/or other demethylated curcuminoids either alone or in combination together with nonionic surfactant, cosolvent and optionally cyclodextrin along with suitable ophthalmic excipients.

Further, a composition of the present invention for use in a method for treatment or prevention of a number of ocular diseases or disorders is disclosed in the present invention. In particular, the composition of the invention may be used to ameliorate diabetic retinopathy, macular degeneration, and other retinopathies, as well as dry eye, uvetis, presbyopia, glaucoma, blepharitis and rosacea of the eye. The composition of the invention may be administered in an opthamologically acceptable carrier or diluent along with natural curcuminoids, such as curcumin, bisdemethoxycurcumin demethoxycurcumin and synthetically derived bis-o-demethyl curcumin and/or other demethylated curcuminoids either alone or in combiantions in a therapeutically effective amount to prevent, retard the development or reduce the symptoms of one or more ophthalmic disease conditions.

### Detailed Description of the Invention:

The invention will now be described in detail in connection with certain preferred and optional embodiments, so that various aspects thereof may be more fully understood and appreciated.

As used herein the term 'curcuminoids' means curcumin mixture comprising natural curcuminoids, such as curcumin, bisdemethoxycurcumin demethoxycurcumin and synthetically derived bis-o-demethyl curcumin and/or other demethylated curcuminoids either alone or in combination having antioxidant or anti-inflammatory activity.

As used herein the term 'surfactant' means surface active agent which works by lowering the surface tension of water and enabling solubility of lipophilic compounds. The surfactants are amphiphilic, meaning .they contain a hydrophobic tail and a hydrophilic head. Therefore, these are soluble in both hydrophobic medium and water. The surfactants are of 2 types a)ionic b) nonionic. The current invention involves the use of nonionic surfactants for pharmaceutical purposes. The surfactants used in the current invention can be selected from polysorbate 80 and 20.

Accordingly, in one embodiment, the instant invention provides an aqueous ophthalmic (topical) eye drop or eye gel formulation comprising one or more natural curcuminoids, such as curcumin, bisdemethoxycurcumin demethoxycurcumin and synthetically derived bis-o-demethyl curcumin and/or other demethylated curcuminoids either alone or in combinations together with nonionic surfactant, cosolvent along with suitable ophthalmic excipients.

In another embodiment, the invention provides an aqueous ophthalmic eye drop or eye gel formulation comprising one or more natural curcuminoids, such as curcumin, bisdemethoxycurcumin demethoxycurcumin and synthetically derived bis-o-demethyl curcumin and/or other demethylated curcuminoids either alone or in combination together with nonionic surfactant, cosolvent and optionally cyclodextrin along with suitable ophthalmic excipients.

Thus, the present invention provides an aqueous ophthalmic (topical) eye drop or eye gel formulation comprising an effective amount of natural/synthetic curcuminoids and an amount of nonionic surfactant and a cosolvent, effective in increasing the bioavailability of the lipophilic curcuminoids, when administered topically to the eye.

The composition of the invention may be administered by an ophthalmic eye drop dispenser comprising an ophthalmic (topical) composition of an effective amount of natural/synthetic curcuminoids, and an amount of nonionic surfactant and a cosolvent, effectively increase the solubility of the natural active in the composition, as compared to its solubility in crystalline/amorphous cyclodextrin. The compositions of this invention are devoid of any side effect and also effective in prolonging the therapeutic effect of the active agent, when administered to the eye.

The novel aspect of the invention comprises drug, which is commonly used spice and coloring agent used in India, is available from wide sources, having potent antioxidant, anti inflammatory action with no side effect.

Accordingly, the present invention provides ophthalmic compositions, especially eye drops and eye gel, where the formulation may optionally comprise cyclodextrin together with one or more other curcuminoids available from nature.

In a preferred embodiment, the active agents that may be used include, but are not limited to, constituents of natural curcumin mixture comprising natural curcuminoids, such as curcumin, bisdemethoxycurcumin demethoxycurcumin and synthetically derived bis-o-demethyl curcumin and/or other demethylated curcuminoids either alone or in combination having antioxidant or anti-inflammatory activity. The concentration of total curcuminoids in the ophthalmic compositions of the present invention may vary from 0.5% to 2.5%w/v. The nonionic surfactant and nonionic cosolvent are present in the amount of 1% each in the ophthalmic solution.

In another preferred embodiment, the cyclodextrin are incorporated in the formulation as colloidal drug carriers such as microspheres and nano particles in micro and submicron range, which due to their size and/or surface charge, possess mucoadhesive properties that results in enhanced drug absorption into the eye. In fact, these systems are incorporated to enhance drug carrier property and topical drug delivery in to eye i.e with a target specific, with more residence time and more bioavailability with better stability.

According to the invention the drug and non-ionic surfactants can be combined with acceptable carriers to formulate a stable ophthalmic preparation with enhanced bioavailability and stability. These carriers include pharmaceutically acceptable excipients and additives known to a person skilled in the art, for example, especially carriers, stabilizers, tonicity enhancing agents buffer substances, preservatives, thickeners, complexing agents and other excipients.

The non-ionic surfactant is not restricted to the following, selected from polysorbate 80 and 20, polyethylene glycol esters, glycerol ethers or mixtures of those compounds.

The co-solvents are selected from polyhydric alcohols such as polyethylene glycol, polypropelene glycol and the like.

The invention further comprises a chelating agent such as disodium salt of EDTA.

The invention may comprise suitable water soluble polymers which may be used in the invention but not restricted to methylcellulose, hydroxyethyl cellulose, hydroxypropyl cellulose, hydroxypropyl methylcellulose, hydroxypropyl ethylcellulose, sodium carboxymethylcellulose, polyvinyl alcohol, polyvinylpyrrolidone, polyethylene glycol, poly(methyl methacrylate), polycarbophil, gelatin, alginate, poly(acrylic acid), polyethylene oxide and chitosan or a derivative thereof.

The stabilizing agent is not restricted to sodium hydrogen sulfite, glycerin, sodium citrate, butyl hydroxyanisole, benzalkonium chloride, edetic acid and pharmaceutically acceptable salts thereof, tocopherol and derivatives thereof, with sodium edetate.

The isotonizing agent used in the invention is not restricted to any specific one like sodium chloride, potassium chloride, D-mannitol, glucose, glycerin, xylitol and propylene.

The thickening agent which may be used but not limited to, any specific one like methylcellulose, ethyl cellulose, hydroxypropyl methylcellulose, polyvinyl pyrrolidone, polyvinyl alcohol, sodium chondroitin sulfate, sodium hyaluronate and chitosan.

The preservatives which may be used include but are not limited to, sodium bisulfite, sodium bisulfate, sodium thiosulfate, benzalkonium chloride, chlorobutanol, thimerosal, phenylmercuric acetate, phenylmercuric nitrate, methylparaben, polyvinyl alcohol and phenylethyl alcohol.

The buffering agents that may be used include, but are not limited to, sodium carbonate, sodium tetraborate, sodium phosphate, sodium acetate, sodium bicarbonate.

Electrolytes such as sodium chloride and potassium chloride may also be included in the formulation.

The ophthalmic aqueous pharmaceutical preparation of the present invention may be charged in an appropriate container for better drug delivery.

The dosage form of the ophthalmic aqueous pharmaceutical preparation of the present invention is not restricted to an eye drop, but may be prepared as eye gel. For instance, the ophthalmic aqueous pharmaceutical suspension preparation of the present invention can widely be used for the treatment of all the anterior and posterior related conditions or ocular diseases.

Furthermore, the ophthalmic aqueous solution preparation of the present invention comprises a non-steroidal drug. Therefore, it may be devoid of any side effect even if it is administered for a prolonged period of time with a low probability of damaging corneal epithelial cells or the conjunctival cells.

Potent curcuminoids such as curcumin, bis-o-demethyl curcumin suppress inflammation by inhibiting edema, fibrin deposition, capillary leakage and phagocytic migration, all key features of the inflammatory response. These curcuminoids prevent the release of prostaglandins, some of which have been identified as mediators of cystoid macular edema. Additionally, curcuminoids including bis-o-demethyl curcumin have been shown to inhibit the expression of vascular endothelial growth factor (VEGF), a cytokine which is a potent promoter of vascular permeability.

The use of curcuminoids to date, by conventional routes of administration, has yielded limited success in treating retinal disorders, including macular edema, largely due to the inability to deliver and maintain adequate quantities of the drug to the posterior segment without resultant toxicity. For example, after usual topical administration of eye drops, only about 1% reaches the anterior segment, and only a fraction of that amount moves into the posterior segment. Although intravitreal injections of drug have been used, the exposure to the drug is very brief as the half-life of the drug within the eye is approximately 3 hours.

A typical eye drop formulation of the present invention comprises an aqueous drug suspension of solid natural curcuminoids, such as curcumin, bisdemethoxycurcumin demethoxycurcumin and synthetically derived bis-o-demethyl curcumin and/or other demethylated curcuminoids either alone or in combinations having antioxidant or anti-inflammatory activity together with nonionic surfactant optionally with cyclodextrin complexes, water-soluble polymers such as hydroxypropyl methylcellulose, and other cellulose derivatives, chelating agent, metal ions (such as magnesium ions) and/or organic salts such as sodium acetate and sodium citrate, where such additives are used to stabilize the system and lend it enhanced mucoadhesive properties.

Having described the invention with reference to certain preferred embodiments, other embodiments will become apparent to one skilled in the art from consideration of the specification. The invention is further defined by reference to the following examples describing in detail the methods of preparation and use of the invention.

### Examples:

### Example 1

| **S.No** | **Ingredients** | **Concentration** |
|---|---|---|
| 1 | Natural/synthetic curcuminoid | 20-2500mg |
| 2 | Poly Ethylene Glycol | 0.7g-1g |
| 4 | Tween 80 | 0.7g-1g |
| 6 | Hydroxy Propyl Methyl Cellulose | 0.1g |
| 7 | Benzalkonium Chloride | 20mg |
| 8 | Ethylene Diamine Tetra Acetic Acid (disodium salt) | 100mg |
| 9 | Sodium tetraborate (0.1M) | 3.81g |
| 10 | Boric acid (1M) | 6.8g |
| 11 | NaCl | 0.795g |
| 12 | Sterile water for Injection | 100ml |

### Example 2

| **S.No** | **Ingredients** | **Concentration** |
|---|---|---|
| 1 | Natural/synthetic Curcuminoid | 20-2500mg |
| 2 | Poly Ethylene Glycol | 0.7g-1g |
| 4 | Tween 20 | 0.7g-1g |
| 6 | Hydroxy Propyl Methyl Cellulose | 0.1g |
| 7 | Benzalkonium Chloride | 20mg |
| 8 | Ethylene Diamine Tetra Acetic Acid (disodium salt) | 100m |
| 9 | Sodium tetraborate (0.1M) | 3.81g |
| 10 | Boric acid (1M) | 6.8g |
| 11 | NaCl | 0.795g |
| 12 | Sterile water for Injection | 100ml |

### Example 3

| **S.No** | **Ingredients** | **Concentration** |
|---|---|---|
| 1 | Natural/synthetic Curcuminoid | 20-2500mg |
| 2 | Poly Ethylene Glycol | 0.7g-1g |
| 4 | Octoxynol | 0.7g-1g |
| 6 | Hydroxy Propyl Methyl Cellulose | 0.1g |
| 7 | Benzalkonium Chloride | 20mg |
| 8 | Ethylene Diamine Tetra Acetic Acid (disodium salt) | 100mg |
| 9 | Sodium tetraborate (0.1M) | 3.81g |
| 10 | Boric acid (1M) | 6.8g |
| 11 | NaCl | 0.795g |
| 12 | Sterile water for Injection | 100ml |

### Example 4

| **S.No** | **Ingredients** | **Concentration** |
|---|---|---|
| 1 | Natural/synthetic Curcumin | 20-2500mg |
| 2 | Poly Ethylene Glycol | 0.7g-1g |
| 4 | Poloxamers | 0.7g-1g |
| 6 | Hydroxy Propyl Methyl Cellulose | 0.1g |
| 7 | Benzalkonium Chloride | 20mg |
| 8 | Ethylene Diamine Tetra Acetic Acid (disodium salt) | 100mg |
| 9 | Sodium tetraborate (0.1M) | 3.81g |
| 10 | Boric acid (1M) | 6.8g |
| 11 | NaCl | 0.795g |
| 12 | Sterile water for Injection | 100ml |

### Example 5:

A typical ophthalmic formulation of 0.06% (curculife)

| **S.No** | **Ingredients** | **Concentration** |
|---|---|---|
| 1 | Curcumin | 60mg |
| 2 | Poly Ethylene Glycol | 1g |
| 4 | Tween 80 | 1g |
| 6 | Hydroxy Propyl Methyl Cellulose | 0.1g |
| 7 | Benzalkonium Chloride | 20mg |
| 8 | Ethylene Diamine Tetra Acetic Acid (disodium salt) | 100mg |
| 9 | Sodium tetraborate (0.1M) | 3.81g |
| 10 | Boric acid (1M) | 6.8g |
| 11 | NaCl | 0.795g |
| 12 | Sterile water for Injection | 100ml |

### Method for preparation:

### Preparation of borate buffer:

1) 3.81g of sodium tetraborate was weighed into a beaker and dissolved in 100ml of water.
2) 6.8g of boric acid was weighed into a beaker and dissolved in 100ml of water.
3) pH of sodium tetraborate solution was checked and found to be in the range of 9. This was adjusted to a pH of 7.2 by the addition of boric acid.
4) The buffer thus prepared was used to adjust pH of the formulation.

### Solubulization of curcumin:

1) 60mg curcumin was weighed into a 10ml beaker
2) To this 1 g (765ul) Tween80 and 1g PEG was added and stirred well using a glass rod.
3) Following this the tube was sonicated for 30min or till the curcumin is completely solubulized.
4) A deep red colour solution was formed.

### Preparation of ophthalmic formulation:

1) 100 ml water was placed into a beaker on a magnetic stirrer.
2) HPMC was added and stirred till all the HPMC dissolved.
3) The curcumin + tween 80 solution was added drop by drop into this stirred for 15 mins.
4) To this BAC, EDTA and NaCl was added and stirred till all the contents dissolve completely and a yellow colour clear solution was obtained.
5) pH of the solution was checked and found to be in the range of 5.3-5.4. Thus it was adjusted with the borate buffer to a pH of 6.5.

### Example 6

| **SL. NO.** | **INGREDIENTS** | **RM CODE** | **SPECIFICATION** | **LABEL CLAIM** | **QTY/ BATCH** |
|---|---|---|---|---|---|
| 1. | Hydroxy Propyl Methyl Cellulose | RH-04 | I.P | 0.1 %w/v | 20.0g |
| 2 | Borax | RB-03 | I.P. | -- | 4.670g |
| 3 | Benzalkonium chloride (50%) solution | RB-02 | I.P. | 0.02% w/v | 4.0g |
| 4. | Edetate disodium | RE-01 | I.P. | -- | 20.0g |
| 5. | Sodium chloride | RS-02 | I.P. | -- | 159.1g |
| 6 | Polyethylene glycol -400 | RP-09 | I.P. | -- | 224g |
| 7 | Polysorbate-80 | RT - 02 | I.P. | -- | 212g |
| 8 | Curcumin 98% | RC-07 | I.H.S | 0.04-0.05% w/v | 12.0g |
| 9 | Purified Water qs | | -- | -- | 20.0L |

### Method for preparation:

### Preparation of Solution A:

1. Weighing accurately 20gm of Hydroxypropyl methylcellulose and adding the same to a vessel containing 2L water (10 % of batch size) which is heated to about 90°C and kept over night.
2. Stirring the said solution on the next day until dispersed completely and making the volume to 6L (30 % of batch size) with purified water and stirring the same well to get homogenous dispersion.
3. Filtering the said solution through 2µ prefilter into the filling vessel. This solution is then autoclaved.

### Preparation of Solution B:

1. Collecting 14L purified water, freshly in the manufacturing vessel. Weighing accurately 4.67g of Borax, add to the manufacturing vessel. Stirring until completely dissolved.
2. weighing accurately 4ml of Benzalkonium chloride solution and adding to the manufacturing vessel and stirring until completely dissolved;
3. weighing accurately 20g of Edetate disodium and adding to the manufacturing vessel alongwith stirring until completely dissolved; and
4. weighing accurately 159g of Sodium chloride and adding it to the manufacturing vessel alongwith stirring until completely dissolved.

### Preparation of Solution C/ Nanoemulsification of curcuminoids:

1. Weighing accurately 12g of Curcumin98% and adding it to the glass beaker;
2. measuring accurately 212g of Polysorbate-80 and adding to a glass beaker of suitable size;
3. measuring accurately 224g of Polyethylene glycol 400 and adding it to the beaker; and
4. sonicating the mixture for 30 mins, until completely dissolved.

### Preparation of bulk solution:

1. Adding 'Solution C' with 'Solution B' and stir well;
2. filtering the solution through 0.2 µ filter into the autoclaved filling vessel containing 'Solution A'; and
3. checking the pH which should be between 5.8 - 6.4.

### Example 7

### Particle size distribution data for nanoemulsified curcumin 98% and BDMC:

A Particle size distribution study conducted on the nano-emulsified formulation has further confirmed that the curcumin molecules exist as nano sized particles in the current formulation. This study proves that a curcumin molecule which is originally 374nm in the unformulated form is emulsified into 8-11nm sized particles through a unique process, which has been indicated for this enhanced transport and efficacy of curcumin through our formulation.

The study was conducted using Malvern particle size analyzer. The map of scattering intensity versus angle is the primary source of information used to calculate the particle size. The scattering of particles is accurately predicted by the Mie scattering model allowing accurate sizing across the widest possible dynamic range. During the laser diffraction measurement, particles are passed through a focused laser beam. These particles scatter light at an angle that is inversely proportional to their size. The angular intensity of the scattered light is then measured by a series of photosensitive detectors. The advantage of this study is that there is no sample preparation involved.

The study conducted on the particle size distribution of curcumin 98% and nanoemulsified BDMC and curcumin 98% clearly showed the particle sizes of the nanoemulsified BDMC and curcumin 98% was between 8-11nm which is the ideal size of a nano-emulsified compound when compared to the particle size of unformulated curcumin 98% which was 373nm. This is a clear indication that the process of nanoemulsification followed has effectively transformed the large sized particles to nanosized coated curcuminoid particles. Additional peaks can be observed which is contributed by the high molecular weight excipients used to prepare the formulation. The results obtained demonstrate that the current nanoemulsification process used in the study is simple and effective.

### Example 8:

### Invivo Bioavailability Study conducted on nanoemulsified Bis-o-demethylcurcumin (BDMC):

Bioavailability of the above formulation was studied on A549 cell line of human lung carcinoma fibroblast cell line. These are adherent cells with quick growth capabilities. The cells were cultured in DMEM media until they reached ~90% confluency after which they were split into 12-well plates. When the culture plates become ~90-100% confluent, they were used for the experiment. Normally, a seeding density of 10⁵ cells per well can give rise to ~90% confluent cultures in 3 days time.

The effect of concentration of nanoemulsified Bis-o-demethylcurcumin, in a dose dependant manner on the transport efficiency of the said compound through A549 epithelial cell line was studied.

| Bis-o-demethylcurcumin conc. (nano-moles/mL) | Bis-o-demethylcurcumin conc from std, graph (nano-moles/mL) |
|---|---|
| 4.076 | 0.448 |
| 8.152 | 0.609 |
| 16.304 | 0.795 |
| 32.609 | 1.006 |

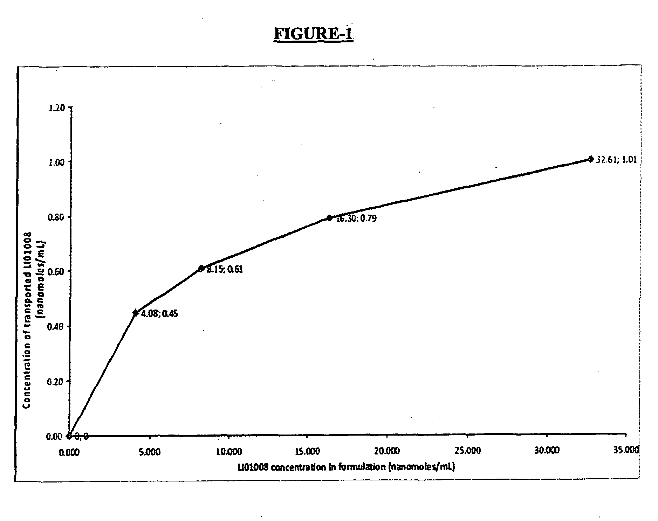

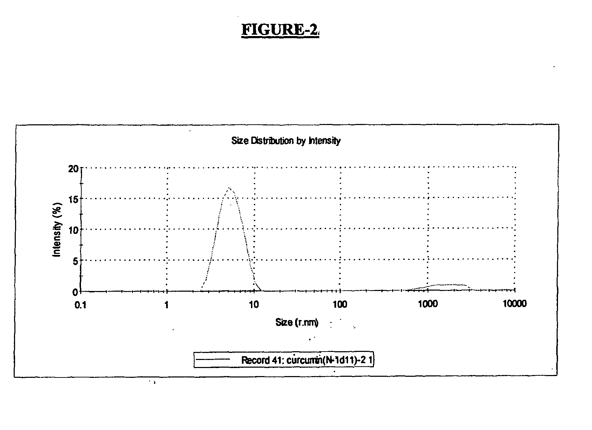

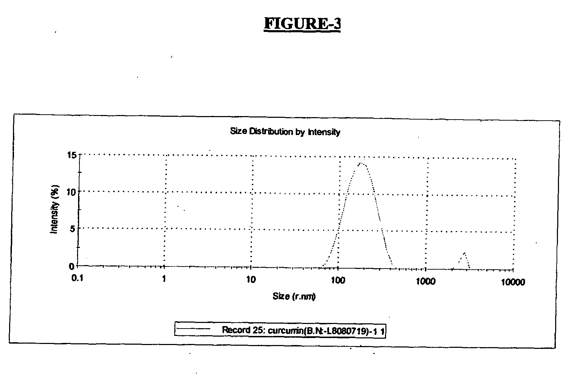

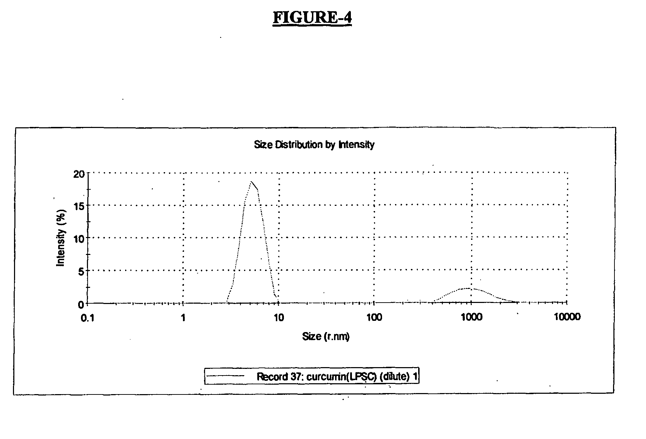

A dose response using varying concentrations of the formulation of the present invention was conducted to study the effect of one of the pharmacokinetic parameter on the transport of Bis-o-demethylcurcumin into the A549 cells. The dose response curve indicated by the graph (fig.1) representing the study undertaken indicates that there is definitely a concentration dependent increase in the transport of nanoemulsified Bis-o-demethylcurcumin across A549 cell monolayers and hence, it can be inferred that concentration is definitely a key parameter in the transport of nanoemulsified Bis-o-demethylcurcumin.

The transport of the above formulation was further studied in vivo through the ocular tissue of rabbits into the vitreous fluid. The vitreous fluid was collected and analyzed using HPLC. The compound was transported in detectable amounts and estimated at different time intervals.

### Example 9:

### In vivo anti-cataract efficacy of Curcumin 98% ophthalmic formulation:

An in vivo efficacy experiment on the anti-cataract effect of the formulation indicates that the compound is able to permeate through the ocular barriers and reach the retina and prevents the cataract formation in rats.

Study was conducted to determine the anti-cataract effect of Curcumin 98% ophthalmic formulation to develop a safe and effective treatment for the prevention or delay the cataract. Animals were grouped into Group I (control), Group II (sodium selenite) and Group III (treatment).

On 10th day of postnatal period, Curcumin 98% ophthalmic formulation was instilled topically to the eyes of group III animals only. Two hours later Sodium Selenite (25 µM/kg.bw of 5ml/ kg.bw) was injected subcutaneously to both group II and III animals, thereafter 5 µl of LP002-09 was instilled topically to the eyes of group III animals only. The Curcumin 98% ophthalmic formulation was instilled daily thrice at an interval of 4 hours for a period of one week, whereas the group I and II animals were left undisturbed through out the dosing period. During instillation of the Curcumin 98% ophthalmic formulation the eyelids of the rat pups were opened gently and carefully and held approximately one minute to prevent overflowing of Curcumin 98% ophthalmic formulation.

All animals were observed individually through out the observation period for any clinical signs and mortality. On the examination day i.e. when the pups first opened their eyes, the development of cataract was observed by the presence or absence of opacity by dilating the pupil with 0.8 % tropicamide.

The cataract was scored on a scale of 0 to 4 according to procedure described by Muranov et al., (2004). Grade 0 was a normal clear lens, Grade 1 was a subcapsular opacity; Grade 2 was nuclear cataract, Grade 3 was strong nuclear cataract; Grade 4 was dense opacity involving entire lens and the results have been presented in below table 1

**Table 1**

| **Group No.** | **No. of Eyes** | **Cataract Type** | | | | | | |
|---|---|---|---|---|---|---|---|---|
| | | **No Opacity** | **Subcapsular opacity** | **Nuclear cataract** | **Strong Nuclear cataract** | **Dense opacity involving entire lens** | **Total Percentage of cataract** | **Grade (Mean ± S.D.)** |
| | | **Score** | | | | | | |
| | | **0** | **1** | **2** | **3** | **4** | | |
| **I** | **20** | **20** | **0** | **0** | **0** | **0** | **0** | **00.0 ± 00.0** |
| **Control** | | | | | | | | |
| **II** | **20** | **0** | **0** | **2** | **4** | **14** | **100** | **3.6 ±0.68** |
| **Sodium Selenite Treated** | | | | | | | | |
| **III** | **20** | **11** | **5** | **2** | **2** | **0** | **45** | **0.75±1.02 *** |
| **LP002-09 treated** | | | | | | | | |

| | | | | | | | | |
|---|---|---|---|---|---|---|---|---|
| ***Highly significant (P<0.0001*) at 5% level. vs. Sodium Selenite treated** | | | | | | | | |

### RESULTS:

None of the animals exhibited clinical signs of toxicity and mortality throughout the experimental period. All the eyes of group I (control) animals were found to be clear. In group II animals in which Sodium Selenite was injected developed dense opacity type cataract in 70% of eyes, strong nuclear cataract in 20 % eyes and nuclear cataract in 10 % of eyes where as in Group III (treatment) animals no cataract was observed in 55 % (11 eyes) of the eyes, strong nuclear cataract in 10 % (2 eyes), nuclear cataract in 10 % (2 eyes) and subcapsular opacity in 25 % (5 eyes) of eyes.

Under these experimental conditions, thus it is concluded that the instillation of Curcumin 98% ophthalmic formulation into the eye of Wistar rat pups effectively reduces the effect of selenite-induced cataract. Hence the experiments conclude that curcumin is transported better through the formulation of the present invention, thereby leading to enhanced bioavailability resulting in improved efficacy.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. An aqueous ophthalmic composition comprising;
a) natural or synthetic curcuminoid(s) either alone or in combination in an amount of 0.02 to 2.5%w/v wherein the particle size of curcuminoid is present in the range of 8-11 nm (nanometer);
b) nonionic surfactant(s) in an amount of 1%w/v;
c) and a polyhydric alcohol co-solvent(s) in an amount of 1%w/v along with pharmaceutically acceptable ophthalmic excipients, for increasing the bioavailability of the active compounds when administered topically to the eye.

2. The aqueous ophthalmic composition as claimed in Claim 1, wherein said composition is in the form of an eye drop or eye gel.

3. The aqueous ophthalmic composition as claimed in Claim 1 or Claim 2, wherein said natural curcuminoids are selected from curcumin, bisdemethoxycurcumin, demethoxycurcumin or mixtures thereof.

4. The aqueous ophthalmic composition as claimed in any one of Claims 1 to 3, wherein said synthetic curcuminoids are selected from synthetically derived bis-o-demethyl curcumin and/or other demethylated curcuminoids or mixtures thereof.

5. The aqueous ophthalmic composition as claimed in any one of Claims 1 to 4, wherein the nonionic surfactant(s) is selected from polysorbate 80 and 20, polyethylene glycol esters, glycerol ethers or combinations thereof, and/or wherein the polyhydric alcohols is selected from polyethylene glycol or polypropylene glycol or mixtures thereof.

6. The aqueous ophthalmic composition as claimed in any one of Claims 1 to 5, wherein suitable ophthalmic excipients are selected from:
polymers, for example water soluble polymers such as methylcellulose, hydroxyethyl cellulose, hydroxypropyl cellulose, hydroxypropyl methylcellulose, hydroxypropyl ethylcellulose, sodium carboxymethylcellulose, polyvinyl alcohol, polyvinylpyrrolidone, polyethylene glycol, poly(methyl methacrylate), polycarbophil, gelatin, alginate, poly(acrylic acid), polyethylene oxide, chitosan, or mixtures thereof;
chelating agents, for example disodium salt of EDTA;
stabilizers, for example sodium hydrogen sulfite, glycerin, sodium citrate, butyl hydroxyanisole, benzalkonium chloride, edetic acid and pharmaceutically acceptable salts thereof, tocopherol, with sodium edentate, or mixtures thereof;
tonicity enhancing agents, for example sodium chloride, potassium chloride, D-mannitol, glucose, glycerin, xylitol, propylene or mixtures thereof;
buffer substances, for example sodium carbonate, sodium tetraborate, sodium phosphate, sodium acetate, sodium bicarbonate or mixtures thereof;
preservatives, for example sodium bisulfite, sodium bisulfate, sodium thiosulfate, benzalkonium chloride, chlorobutanol, thimerosal, phenylmercuric acetate, phenylmercuric nitrate, methylparaben, polyvinyl alcohol, phenylethyl alcohol or mixtures thereof;
thickeners, for example methylcellulose, ethyl cellulose, hydroxypropyl methylcellulose, polyvinyl pyrrolidone, polyvinyl alcohol, sodium chondroitin sulfate, sodium hyaluronate, chitosan or mixtures thereof; complexing agents; or
mixtures thereof.

7. The aqueous ophthalmic composition as claimed in any one of Claims 1 to 6, further comprising cyclodextrin as colloidal drug carriers such as microspheres and nano particles in micro and submicron range.

8. The aqueous ophthalmic composition as claimed in any one of Claims 1 to 7 for use in the prevention and treatment of ocular disease conditions including Behcet's disease, retinal disorders, diabetic retinopathy, cataract, conjunctival diseases, corneal diseases including macular degeneration and glaucoma.

## Patentansprüche

1. Wässrige ophthalmische Zusammensetzung, die Folgendes umfasst:
a) natürliches/natürliche oder synthetisches/synthetische Kurkuminoid/e entweder allein oder in Kombination in einer Menge von 0,02 bis 2,5 % m/V, worin die Partikelgröße von Kurkuminoid im Bereich von 8-11 nm (Nanometer) liegt;
b) nichtionisches/nichtionische Tensid/e in einer Menge von 1 % m/V;
c) und (ein) Colösungsmittel von mehrwertigem Alkohol in einer Menge von 1 % m/V zusammen mit pharmazeutisch verträglichen ophthalmischen Hilfsstoffen zur Erhöhung der Bioverfügbarkeit der aktiven Verbindungen bei topischer Verabreichung an das Auge.

2. Wässrige ophthalmische Zusammensetzung nach Anspruch 1, worin die genannte Zusammensetzung in Form von Augentropfen oder Augengel vorliegt.

3. Wässrige ophthalmische Zusammensetzung nach Anspruch 1 oder Anspruch 2, worin die genannten natürlichen Kurkuminoide aus Kurkumin, Bisdemethoxykurkumin, Demethoxykurkumin oder Mischungen davon ausgewählt sind.

4. Wässrige ophthalmische Zusammensetzung nach einem der Ansprüche 1 bis 3, worin die genannten synthetischen Kurkuminoide aus synthetisch derivatisiertem Bis-o-demethylkurkumin und/oder anderen demethylierten Kurkuminoiden oder Mischungen davon ausgewählt sind.

5. Wässrige ophthalmische Zusammensetzung nach einem der Ansprüche 1 bis 4, worin das/die nichtionische/n Tensid/e aus Polysorbat 80 und 20, Polyethylenglycolestern, Glycerolethern oder Kombinationen davon ausgewählt ist/sind und/oder worin die mehrwertigen Alkohole aus Polyethylenglycol oder Polypropylenglycol oder Mischungen davon ausgewählt sind.

6. Wässrige ophthalmische Zusammensetzung nach einem der Ansprüche 1 bis 5, worin geeignete ophthalmische Hilfsstoffe aus Folgenden ausgewählt sind:
Polymeren, beispielsweise wasserlöslichen Polymeren, wie zum Beispiel Methylcellulose, Hydroxyethylcellulose, Hydroxypropylcellulose, Hydroxypropylmethylcellulose, Hydroxypropylethylcellulose, NatriumCarboxymethylcellulose, Polyvinylalkohol, Polyvinylpyrrolidon, Polyethylenglycol, Polymethylmethacrylat, Polycarbophil, Gelatine, Alginat, Polyacrylsäure, Polyethylenoxid, Chitosan oder Mischungen davon;
Chelatbildnern, beispielsweise Dinatriumsalz von EDTA;
Stabilisatoren, beispielsweise Natriumhydrogensulfit, Glycerin, Natriumcitrat, Butylhydroxyanisol, Benzalkoniumchlorid, Edetinsäure und pharmazeutisch verträglichen Salzen davon, Tocopherol, mit Natriumedetat oder Mischungen davon;
die Tonizität erhöhenden Mitteln, beispielsweise Natriumchlorid, Kaliumchlorid, D-Mannitol, Glucose, Glycerin, Xylitol, Propylen oder Mischungen davon;
Puffersubstanzen, beispielsweise Natriumcarbonat, Natriumtetraborat, Natriumphosphat, Natriumacetat, Natriumbicarbonat oder Mischungen davon;
Konservierungsmitteln, beispielsweise Natriumbisulfit, Natriumbisulfat, Natriumthiosulfat, Benzalkoniumchlorid, Chlorbutanol, Thimerosal, Phenylquecksilberacetat, Phenylquecksilbernitrat, Methylparaben, Polyvinylalkohol, Phenylethylalkohol oder Mischungen davon;
Verdickungsmitteln, beispielsweise Methylcellulose, Ethylcellulose, Hydroxypropylmethylcellulose, Polyvinylpyrrolidon, Polyvinylalkohol, Natriumchondroitinsulfat, Natriumhyaluronat, Chitosan oder Mischungen davon;
Komplexierungsmitteln; oder
Mischungen davon.

7. Wässrige ophthalmische Zusammensetzung nach einem der Ansprüche 1 bis 6, die weiter Cyclodextrin als kolloidalen Arzneistoffträger umfasst, wie zum Beispiel Mikrosphären und Nanopartikel im Mikro- und Submikronbereich.

8. Wässrige ophthalmische Zusammensetzung nach einem der Ansprüche 1 bis 7 für die Verwendung bei der Prävention und Behandlung von Augenerkrankungszuständen, die Folgende einschließen: Behcet-Krankheit, Netzhauterkrankungen, diabetische Retinopathie, Katarakt, Bindehauterkrankungen, Hornhauterkrankungen, einschließlich Makuladegeneration und Glaukom.

## Revendications

1. Composition ophtalmique aqueuse comprenant :
a) un ou des curcuminoïde(s) naturel(s) ou synthétique(s) présent(s) seul(s) ou en combinaison à une quantité de 0,02 à 2,5 % p/v, la taille des particules du curcuminoïde étant dans la plage de 8-11 nm (nanomètre) ;
b) un ou des agent(s) tensio-actif(s) non ionique(s) à une quantité de 1 % p/v ;
c) et un ou des alcool(s) polyhydrique(s) servant de co-solvant(s) à une quantité de 1 % p/v ainsi que des excipients ophtalmiques pharmaceutiquement acceptables, pour augmenter la biodisponibilité des composés actifs lors d'une administration topique dans l'oeil.

2. Composition ophtalmique aqueuse selon la revendication 1, dans laquelle ladite composition est sous la forme d'un collyre ou d'un gel oculaire.

3. Composition ophtalmique aqueuse selon la revendication 1 ou la revendication 2, dans laquelle lesdits curcuminoïdes naturels sont sélectionnés parmi la curcumine, la bisdéméthoxycurcumine, la déméthoxycurcumine ou des mélanges de celles-ci.

4. Composition ophtalmique aqueuse selon l'une quelconque des revendications 1 à 3, dans laquelle lesdits curcuminoïdes synthétiques sont sélectionnés parmi une bis-o-déméthylcurcumine et/ou d'autres curcuminoïdes déméthylés d'origine synthétique ou des mélanges de ceux-ci.

5. Composition ophtalmique aqueuse selon l'une quelconque des revendications 1 à 4, dans laquelle le ou les agent(s) tensio-actif(s) non ionique(s) est/sont sélectionné(s) parmi le polysorbate 80 et 20, des esters de polyéthylène-glycol, des éthers de glycérol ou des combinaisons de ceux-ci, et/ou dans laquelle les alcools polyhydriques sont sélectionnés parmi le polyéthylène-glycol ou le polypropylène-glycol ou des mélanges de ceux-ci.

6. Composition ophtalmique aqueuse selon l'une quelconque des revendications 1 à 5, dans laquelle les excipients ophtalmiques appropriés sont sélectionnés parmi :
des polymères, par exemple des polymères hydrosolubles comme la méthylcellulose, l'hydroxyéthylcellulose, l'hydroxypropylcellulose, l'hydroxypropylméthylcellulose, l'hydroxypropyléthylcellulose, la carboxyméthylcellulose sodique, un alcool polyvinylique, la polyvinylpyrrolidone, le polyéthylène-glycol, un poly(méthacrylate de méthyle), un polycarbophile, la gélatine, l'alginate, un poly(acide acrylique), un oxyde de polyéthylène, le chitosane ou des mélanges de ceux-ci ;
des agents chélatants, par exemple le sel disodique de l'EDTA ;
des stabilisants, par exemple l'hydrogénosulfite de sodium, la glycérine, le citrate de sodium, l'hydroxyanisole butylique, le chlorure de benzalkonium, l'acide édétique et
les sels pharmaceutiquement acceptables de celui-ci, le tocophérol et les dérivés de celui-ci avec l'édétate de sodium ou des mélanges de ceux-ci ;
des agents augmentant la tonicité, par exemple le chlorure de sodium, le chlorure de potassium, le D-mannitol, le glucose, la glycérine, le xylitol, le propylène ou des mélanges de ceux-ci ;
des substances tampons, par exemple le carbonate de sodium, le tétraborate de sodium, le phosphate de sodium, l'acétate de sodium, le bicarbonate de sodium ou des mélanges de ceux-ci ;
des agents conservateurs, par exemple le bisulfite de sodium, le bisulfate de sodium, le thiosulfate de sodium, le chlorure de benzalkonium, le chlorobutanol, le thimérosal, l'acétate de phénylmercure, le nitrate de phénylmercure, le méthylparabène, un alcool polyvinylique, l'alcool phényléthylique ou des mélanges de ceux-ci ;
des épaississants, par exemple la méthylcellulose, l'éthylcellulose, l'hydroxypropylméthylcellulose, la polyvinylpyrrolidone, un alcool polyvinylique, le sulfate de chondroïtine sodique, l'hyaluronate de sodium, le chitosane ou des mélanges de ceux-ci ;
des agents complexants ; ou
des mélanges de ceux-ci.

7. Composition ophtalmique aqueuse selon l'une quelconque des revendications 1 à 6, qui comprend également la cyclodextrine sous forme de véhicules médicamenteux colloïdaux tels que des microsphères et des nanoparticules de dimensions micro- et sous-microniques.

8. Composition ophtalmique aqueuse selon l'une quelconque des revendications 1 à 7 pour une utilisation dans la prévention et le traitement de maladies oculaires, y compris de la maladie de Behçet, d'affections rétiniennes, de la rétinopathie diabétique, de la cataracte, de maladies de la conjonctive, de maladies de la cornée dont la dégénérescence maculaire, et du glaucome.
